# EUROPEAN PATENT APPLICATION

(11) **EP 3 400 967 A1**
(43) Date of publication of application: **14.11.2018**
(21) Application number: 16883768.0
(22) Date of filing: 16.12.2016
(51) Int. Cl.: A61L 2/02

(54) **STERILIZATION METHOD AND STERILIZATION MECHANISM**

(30) Priority: 05.01.2016 JP 2016000633
(71) Applicant: Suntory Holdings Limited, Osaka-shi, Osaka 530-8203 (JP)
(72) Inventor: TAKEMASA, Ryo, Kyoto 619-0284 (JP); HIGASHIYAMA, Kenichi, Kyoto 619-0284 (JP)
(74) Representative: Vossius & Partner Patentanwälte Rechtsanwälte mbB
(86) International application number: PCT/JP2016/087593
(87) International publication number: WO 2017/119268

(57) **Abstract**

To perform sterilization using a sterilization member (1) that has a treatment surface (1A) that is a surface on which a plurality of protrusions each having a leading end that is thinner than a diameter of a microorganism that is to be killed are provided, a sterilization member on which each protrusion has a height that is greater than or equal to half the diameter of the microorganism that is to be killed is used as the sterilization member (1), and a fluid (5) that contains the microorganism that is to be killed is caused to strike the treatment surface (1A) to kill the microorganism that is to be killed.

## Description

### Technical Field

The present invention relates to a sterilization method and a sterilization mechanism that employ a sterilization member that has a treatment surface that is a surface on which a plurality of protrusions are provided.

### Background Art

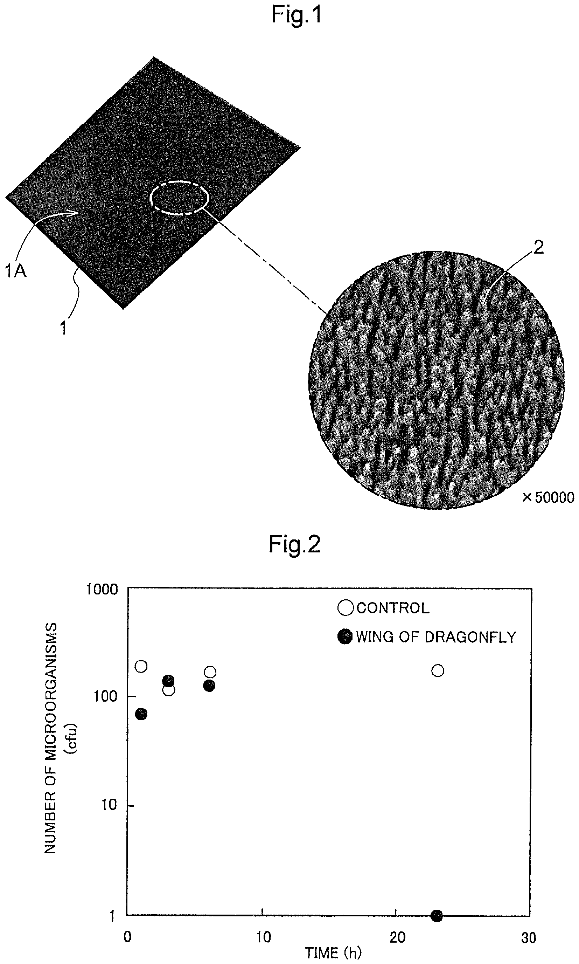

The paper "Bactericidal activity of black silicon" (Non-Patent Document 1) written by Elena and other twelve authors reports that the wings of a dragonfly and black silicon, which have a surface structure provided with a large number of protrusions that each have a nanoscale thickness (e.g. FIG. 1), have a sterilization effect on various species of bacteria. Specifically, it has been found that the number of bacteria in suspension in a liquid is reduced by a wing of a dragonfly or a sheet of black silicon left in the liquid as it is. However, the technique of sterilization that employs a sterilization member such as a wing of a dragonfly or black silicon, which has a surface structure provided with a plurality of protrusions on the surface, still has room for improvement in terms of practical use.

### Prior Art Documents

### Non-Patent Documents

Non-Patent Document 1: Elena P. Ivanova and other twelve authors, "Bactericidal activity of black silicon", NATURE COMMUNICATIONS, 4:2838, DOI: 10.1038/ncomms3838

### Summary of the Invention

### Problem to be Solved by the Invention

It is desired to realize a sterilization method and a sterilization mechanism that make it possible to preferably perform sterilization using the above-described sterilization member.

### Means for Solving Problem

A sterilization method according to the present invention is
a sterilization method that employs a sterilization member that has a treatment surface that is a surface on which a plurality of protrusions are provided, each protrusion having a leading end that is thinner than a diameter of a microorganism that is to be killed,
the sterilization member thus employed being a sterilization member on which each protrusion has a height that is greater than or equal to half the diameter of the microorganism that is to be killed,
the sterilization method including:
causing a fluid that contains the microorganism that is to be killed to strike the treatment surface of the sterilization member to kill the microorganism that is to be killed.

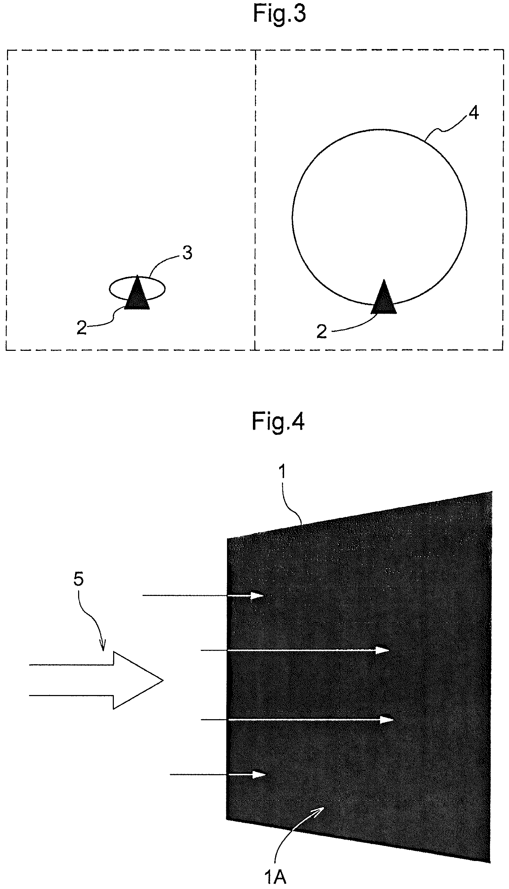

The inventors have found that the sterilization effect of the sterilization member on each species of microorganisms is linked to the dimensions of the protrusions relative to the diameter of the species of microorganisms. Specifically, the inventors have found that a preferable sterilization effect is produced when the protrusions of the sterilization member have a height that is at least half the diameter of the species of microorganisms. Based on this phenomenon, the inventors have conceived of the idea that at least one factor of the sterilization effect produced by such a sterilization member is that when microorganisms are brought into contact with the surface on which a large number of protrusions are provided, the microorganisms are affected by the physical action of the protrusions (for example, as shown in FIG. 3, a microorganism is pierced by a protrusion). Furthermore, based on this conception, the inventors have conceived of the possibility that the physical action of the protrusions can be enhanced and the sterilization effect can be improved by applying an external force to either the microorganisms or the sterilization member so that the microorganisms and the treatment surface strike each other, instead of simply bringing the microorganisms into contact with the treatment surface. With the above-described configuration, it is possible to effectively perform sterilization by employing the preferable technique that have found by the inventors, i.e. causing a fluid that contains the microorganism that are to be killed to strike the treatment surface of the sterilization member, using, as the sterilization member, a sterilization member on which each protrusion has a height that is greater than or equal to half the diameter of each of the microorganisms that are to be killed.

The following describes preferable aspects of a sterilization method according to the present invention. However, note that the examples of preferable aspects described below are not intended to limit the scope of the present invention.

In one aspect, it is preferable that the fluid that contains the microorganism that is to be killed is caused to strike the treatment surface of the sterilization member to kill the microorganism that is to be killed, by causing the fluid to flow toward the treatment surface, with the sterilization member being fixed.

With this configuration, it is possible to effectively cause the microorganism that is to be killed, to strike the treatment surface of the sterilization member, by causing the fluid to flow toward the treatment surface, and therefore it is possible to more effectively perform sterilization.

In one aspect, it is preferable that a flow of the fluid is accelerated toward the treatment surface at a position that is upstream of the treatment surface in a direction in which the fluid flows.

In order to enhance the physical action of the protrusions, it is possible to conceive of the idea of increasing the flow velocity of the fluid at the time the microorganisms and the treatment surface strike each other so that the microorganisms and the treatment surface strike each other with a greater force. With the above-described configuration, the flow velocity of the fluid is increased only in a section that is upstream of the treatment surface. Therefore, compared to increasing the flow velocity overall, such a configuration requires less energy to increase the flow velocity at the time the microorganisms and the treatment surface strike each other, and realizes efficient operations.

In one aspect of the present embodiment, it is preferable that a pressure that is applied from the fluid to the treatment surface when the fluid and the treatment surface strike each other is greater than or equal to 10 MPa.

The inventors have also found that the sterilization effect of the sterilization member can be further effectively improved if a pressure that is applied from the fluid to the treatment surface when the fluid and the treatment surface strike each other is greater than or equal to 10 MPa. The above-described configuration employs the preferable technique that have found by the inventors, and therefore it is possible to more effectively perform sterilization.

A sterilization mechanism according to the present invention is
a sterilization mechanism that employs a sterilization member that has a treatment surface that is a surface on which a plurality of protrusions are provided, each of the plurality of protrusions having a leading end that is thinner than a diameter of a microorganism that is to be killed,
the sterilization member being a member on which each protrusion has a height that is greater than or equal to half the diameter of the microorganism that is to be killed,
the sterilization mechanism comprising:
a flow channel through which a fluid that contains the microorganism that is to be killed flows,
wherein the sterilization member is installed in a section of the flow channel, and
the sterilization mechanism further includes a flow producing portion that produces a flow of the fluid toward the treatment surface of the sterilization member.

With this configuration, it is possible to achieve the same operational effect as the above-described sterilization method according to the present invention.

In one aspect, it is preferable that the sterilization mechanism further includes: an acceleration mechanism that is located upstream of the treatment surface in a direction in which the fluid flows, and accelerates the flow of the fluid toward the treatment surface.

Compared to increasing the flow velocity overall, such a configuration requires less energy to increase the flow velocity at the time the microorganisms and the treatment surface strike each other, and realizes efficient operations.

### Brief Description of the Drawings

FIG. 1 shows a perspective view and an enlarged view of a sterilization member according to the present invention.
FIG. 2 is a graph showing an example of a sterilization effect of a wing of a dragonfly on bacterial vegetative cells.
FIG. 3 is a conceptual diagram schematically showing action of a protrusion on a yeast cell.
FIG. 4 is a diagram showing an example of a sterilization method according to the present invention.
FIG. 5 is a cross-sectional view showing a homogenizer that is an example of a sterilization mechanism according to the present invention.

### Best Mode for Carrying out the Invention

The following describes a sterilization method and a sterilization mechanism according to the present invention with reference to the drawings. The sterilization method and the sterilization mechanism according to the present embodiment employ a sterilization member 1 that is made of a metallic or non-metallic material and that has a treatment surface 1A that is a surface on which a plurality of protrusions 2 are provided, each protrusion having a leading end that is thinner than a diameter of microorganisms that are to be killed. As the sterilization member 1, a member on which each protrusion 2 has a height that is greater than or equal to half the diameter of the microorganisms that are to be killed is used, and a fluid that contains the microorganisms that are to be killed is caused to strike the treatment surface 1A of the sterilization member 1 to kill the microorganisms that are to be killed. Thus, sterilization can be preferably performed. The following describes the sterilization method and the sterilization mechanism according to the present embodiment in detail.

First, the sterilization member 1 employed in the sterilization method and the sterilization mechanism will be described. The sterilization method and the sterilization mechanism according to the present embodiment employ a sheet-shaped sterilization member 1 that has, as shown in FIG. 1, a treatment surface 1A that is a surface on which a plurality of protrusions 2 are provided, each protrusion 2 having a leading end that is thinner than a diameter of microorganisms that are to be killed (e.g. a leading end that has a nanoscale thickness that is less than or equal to 1 µm). Such a sterilization member 1 may be selected from among existing members, or manufactured so as to be suitable for microorganisms that are to be killed, depending on what microorganisms are to be killed. For example, a wing of a dragonfly or black silicon can be used as a sterilization member 1 for killing bacterial vegetative cells, which are examples of microorganisms.

Also, in the present embodiment, a member on which each protrusion 2 has a height that is greater than or equal to half the diameter of the microorganisms that are to be killed, is particularly employed as the sterilization member 1. This is because such a configuration produces preferable sterilization effect. The following describes this fact with reference to FIG. 2. FIG. 2 shows a sterilization effect over time in a case where a liquid that contains bacterial vegetative cells was dripped onto a wing of a dragonfly, which serves as a sterilization member 1 that has protrusions 2 each having a leading end that has an average thickness of approximately 0.05 µm and an average height of approximately 0.5 µm (indicated by "WING OF DRAGONFLY" in the drawing), and a case where the liquid that contains bacterial vegetative cells was left as it was without being dripped (indicated by "CONTROL" in the drawing). The result of comparison between these cases confirms that a sterilization effect was produced in the case where the liquid was dripped onto the sterilization member 1. Also, the results of observation in a case where a liquid that contains yeast cells (each having a diameter of approximately 3 µm) was dripped onto the sterilization member 1 and in a case where the liquid containing yeast cells was left as it was without being dripped show that no sterilization effect was produced by the sterilization member 1. In this way, a preferable sterilization effect was produced when the protrusions 2 each had a diameter that is greater than or equal to half the diameter of the microorganisms that were to be killed. Furthermore, it more preferable that the protrusions 2 each have a diameter that is greater than or equal to three-fourths of the diameter of the microorganisms that are to be killed. Although a case where the leading ends of the protrusions 2 have an average thickness of approximately 0.05 µm and an average height of approximately 0.5 µm is shown above, the protrusions 2 are not limited in this way, and the thickness and the height of the leading ends of the protrusions 2 may be changed as appropriate, depending on the diameter of the microorganisms that are to be killed.

Considering the fact that the sterilization effect varies depending on the diameter of microorganisms, it is possible to conceive of the possibility that one factor of the sterilization effect produced by the sterilization member 1 is that the microorganisms are affected by the physical action of the protrusions 2. This can be schematically described with reference to FIG. 3. That is, it is possible to conceive of the possibility that even if a yeast cell 4 that has a diameter of approximately 3 µm is affected by a certain physical action of protrusions 2 that have an average thickness of 0.05 µm and an average height of 0.5 µm, e.g. even if a protrusion 2 pierces through the yeast cell 4, the protrusion 2 has little effect on the yeast cell 4, which is significantly larger than the protrusion 2, and cannot kill the yeast cell 4, whereas the protrusions 2 that have an average thickness of 0.05 µm and an average height of 0.5 µm have an effective physical action on a bacterial vegetative cell 3 that has a diameter that is in the range of approximately 0.5 µm to approximately 1.0 µm, and can kill the bacterial vegetative cell 3.

Examples of microorganisms that are to be killed using the sterilization method and the sterilization mechanism according to the present embodiment include bacteria, and fungi such as yeast and molds. Note that a sterilization effect was achieved in both the case of vegetative cells and the case of spores, and there was no influence on the sterilization effect. Also, as the sterilization member 1 employed in the sterilization method and the sterilization mechanism according to the present embodiment, a sterilization member 1 that has protrusions 2 that each have a leading end that is thinner than the diameter of microorganisms that are to be killed and that has a height that is greater than or equal to half the diameter of the microorganisms that are to be killed may be selected as appropriate, or newly manufactured, depending on what microorganisms are to be killed.

In the sterilization method according to the present embodiment, using such a sterilization member 1, a fluid that contains microorganisms that are to be killed and the treatment surface 1A of the sterilization member 1 are caused to strike each other to kill the microorganisms that are to be killed. That is, if microorganisms are killed by being affected by the physical action of the protrusions 2, the physical action of the protrusions 2 can be enhanced and the sterilization effect can be improved by applying an external force to either the microorganisms or the sterilization member 1 so that the microorganisms and the treatment surface 1A strike each other, instead of simply bringing the microorganisms into contact with the treatment surface 1A.

In the sterilization method according to the present embodiment, as one means to cause a fluid that contains microorganisms that are to be killed and the treatment surface 1A of the sterilization member 1 to strike each other to kill the microorganisms that are to be killed, a means in which a fluid 5 that contains the microorganisms that are to be killed is caused to flow toward the treatment surface 1A, with the sterilization member 1 being fixed, as shown in FIG. 4, is employed so that the fluid 5 and the treatment surface 1A strike each other. In this regard, in order to increase the flow velocity of the fluid 5 at the time the microorganisms and the treatment surface 1A strike each other so that the microorganisms and the treatment surface strike each other with a greater force to enhance the physical action of the protrusion 2, it is preferable that the flow of the fluid 5 toward the treatment surface 1A is accelerated at a position that is upstream of the treatment surface 1A in the direction in which the fluid 5 flows. Compared to increasing the flow velocity overall, such a configuration requires less energy to increase the flow velocity at the time the microorganisms and the treatment surface 1A strike each other, and realizes efficient operations.

It is also preferable that the pressure applied from the fluid 5 to the treatment surface 1A when the fluid 5 and the treatment surface 1A strike each other is greater than or equal to 10 MPa.

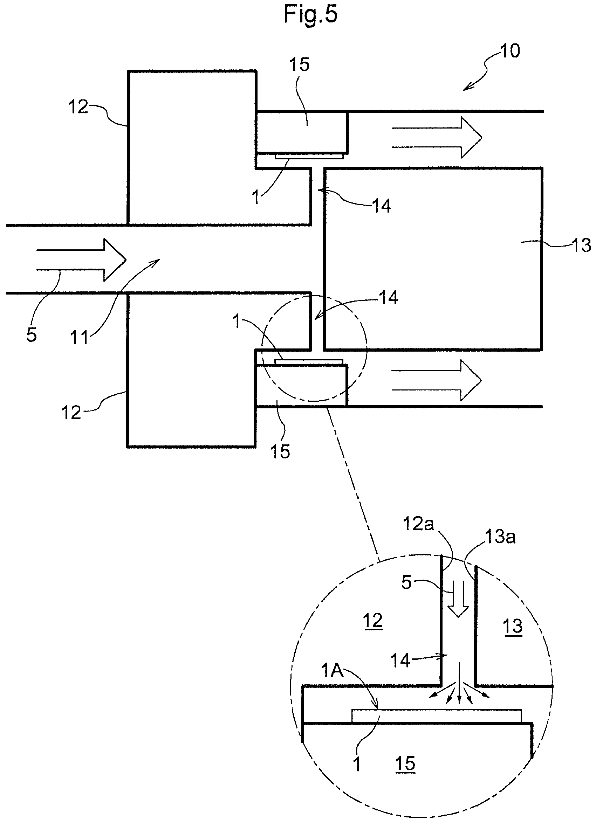

Next, the following describes an example in which the sterilization member 1 is applied to a homogenizer that is an example of a sterilization mechanism that can perform the sterilization method. FIG. 5 shows a typical homogenizer 10 that includes: a valve seat 12 that is ring-shaped and in a central portion of which a main flow channel 11 through which a fluid flows is formed; a valve 13 that has a circular leading end surface 13a that faces a ring-shaped leading end surface 12a of the valve seat 12; and an impact ring 15 that is provided outside a ring-shaped flow channel 14 that is formed between the leading end surface 12a of the valve seat 12 and the leading end surface 13a of the valve 13. In the homogenizer 10, the flow velocity of the fluid 5 pumped through the main flow channel 11 sharply increases when the fluid 5 passes through the ring-shaped flow channel 14 that has a smaller flow channel area than the main flow channel 11, the fluid 5 flowing at the increased flow velocity strikes the impact ring 15, and thus the state of dispersion of substances in the fluid is improved.

Also, in the present embodiment, the sterilization member 1 is installed on the impact ring 15, which is a portion of the flow channel, such that the installation surface 1A faces the ring-shaped flow channel. With such a configuration, the ring-shaped flow channel 14 produces a flow toward the treatment surface of the sterilization member 1, and the fluid 5 is accelerated as a result of passing through the ring-shaped flow channel 14, which brings about a state in which the fluid 5 preferably strikes the treatment surface 1A. That is, the ring-shaped flow channel 14 functions as a flow producing portion that produces a flow of fluid toward the treatment surface 1A of the sterilization member 1, and also functions as an acceleration mechanism that is located upstream of the treatment surface 1A in the direction in which the fluid 5 flows, and accelerates the flow of the fluid 5 toward the treatment surface 1A. Thus, using the homogenizer 10, it is possible to effectively sterilize the fluid 5 that contains microorganisms that are to be killed.

### Other Embodiments

Finally, the following describes other embodiments of the sterilization method and the sterilization mechanism according to the present invention. Note that a configuration disclosed in any one of the following embodiments may be applied in combination with a configuration disclose in another one of the embodiments as long as inconsistencies do not occur.
(1) The embodiment above describes, as one means to cause a fluid that contains microorganisms that are to be killed and the treatment surface 1A of the sterilization member 1 to strike each other to kill the microorganisms that are to be killed, an example configuration in which the fluid 5 that contains the microorganisms that are to be killed is caused to flow toward the treatment surface 1A, with the sterilization member 1 being fixed, as shown in FIG. 4. However, embodiments of the present invention are not limited to this configuration. For example, it is also possible to operate the sterilization member 1, e.g. to cause the sterilization member 1 to strike the fluid 5, or to move the sterilization member 1 in the fluid 5, with the treatment surface 1A facing forward.
(2) The embodiment above describes, as an example of a sterilization mechanism, an example configuration in which the sterilization member 1 is applied to the homogenizer 10. However, embodiments of the present invention are not limited to this configuration. A sterilization mechanism according to the present invention may be any mechanism as long as: each of the protrusions 1A of the sterilization member 1 has a height that is greater than or equal to half the diameter of each of the microorganisms that are to be killed; the mechanism includes a flow channel through which a fluid that contains the microorganisms that are to be killed flows; the sterilization member is installed in a section of the flow channel; and the mechanism includes a flow producing portion that produces a flow of fluid toward the treatment surface 1A of the sterilization member 1. In addition, the flow producing portion is not limited to a portion that regulates the orientation of the flow like the ring-shaped flow channel 14 of the homogenizer 10, and may be a device that produces a flow by itself, such as a pump. The acceleration mechanism is also not limited to a mechanism that employs a structure that accelerates the flow by utilizing a difference in flow area compared to the main flow channel 11, like the ring-shaped flow channel 14 of the homogenizer 10, and may be a mechanism that mechanically accelerates the flow, such as a pump.
(3) Regarding other configurations, the embodiments disclosed in the present description are to be considered as illustrative in all respects, and it is to be understood that the scope of the present invention is not limited to the embodiments. A person skilled in the art would easily understand that the present invention may be modified as appropriate without departing from the spirit of the invention. Therefore, as a matter of course, other embodiments modified without departing from the spirit of the present invention are also encompassed in the scope of the present invention.

### Industrial Applicability

The present invention is applicable to sterilization for killing microorganisms such as bacteria, yeast, and molds.

### Description of Reference Signs

1: Sterilization Member
1A: Treatment surface
2: Protrusion
10: Homogenizer (Sterilization Mechanism)
11: Main Flow Channel (Flow Channel)
14: Ring-shaped Flow Channel (Flow Producing Portion, Acceleration Mechanism)

## Claims

1. A sterilization method that employs a sterilization member that has a treatment surface that is a surface on which a plurality of protrusions are provided, each of the plurality of protrusions having a leading end that is thinner than a diameter of a microorganism that is to be killed,
the sterilization member thus employed being a sterilization member on which each protrusion has a height that is greater than or equal to half the diameter of the microorganism that is to be killed,
the sterilization method comprising:
causing a fluid that contains the microorganism that is to be killed to strike the treatment surface of the sterilization member to kill the microorganism that is to be killed.

2. The sterilization method according to claim 1, wherein the fluid that contains the microorganism that is to be killed is caused to strike the treatment surface of the sterilization member to kill the microorganism that is to be killed, by causing the fluid to flow toward the treatment surface, with the sterilization member being fixed.

3. The sterilization method according to claim 2, further comprising:
accelerating a flow of the fluid toward the treatment surface at a position that is upstream of the treatment surface in a direction in which the fluid flows.

4. The sterilization method according to any one of claims 1 to 3, wherein a pressure that is applied from the fluid to the treatment surface when the fluid and the treatment surface strike each other is greater than or equal to 10 MPa.

5. A sterilization mechanism that employs a sterilization member that has a treatment surface that is a surface on which a plurality of protrusions are provided, each of the plurality of protrusions having a leading end that is thinner than a diameter of a microorganism that is to be killed,
the sterilization member being a sterilization member on which each protrusion has a height that is greater than or equal to half the diameter of the microorganism that is to be killed,
the sterilization mechanism comprising:
a flow channel through which a fluid that contains the microorganism that is to be killed flows,
wherein the sterilization member is installed in a section of the flow channel, and
the sterilization mechanism further comprises a flow producing portion that produces a flow of the fluid toward the treatment surface of the sterilization member.

6. The sterilization mechanism according to claim 5, further comprising:
an acceleration mechanism that is located upstream of the treatment surface in a direction in which the fluid flows, and accelerates the flow of the fluid toward the treatment surface.
